# EUROPEAN PATENT SPECIFICATION

(11) **EP 3 562 504 B1**
(45) Date of publication and mention of the grant of the patent: **19.04.2023**
(21) Application number: 17826321.6
(22) Date of filing: 22.12.2017
(51) Int. Cl.: A61K 39/12, C07K 14/025, C12N 15/62, C12N 15/82, A61P 35/00

(54) **DNA VACCINE COMPRISING A POLYNUCLEOTIDE SEQUENCE ENCODING A FUSION PROTEIN ENCOMPASSING A PLANT PROTEIN SIGNAL SEQUENCE**
DNA IMPFSTOFF ENTHALTEND EIN POLYNUKLEOTID, WELCHES EIN FUSIONSPROTEIN KODIERT, DASS EINE PFLANZENPROTEINSIGNALSEQUENZ ENTHÄLT
VACCIN À ADN COMPRENANT UN POLYNUCLÉOTIDE CODANT POUR UNE PROTÉINE DE FUSION QUI COMPREND UNE SÉQUENCE SIGNAL DE PROTÉINE VÉGÉTALE

(30) Priority: 29.12.2016 IT 201600131935
(43) Date of publication of application: 06.11.2019
(73) Proprietor: ENEA - Agenzia Nazionale per le Nuove Tecnologie, l'Energia e lo Sviluppo Economico Sostenibile, 00196 Roma (RM) (IT); Istituti Fisioterapici Ospitalieri, 00128 Roma (IT)
(72) Inventor: FRANCONI, Rosella, 00196 Roma (IT); MASSA, Silvia, 00196 Roma (IT); VENUTI, Aldo, 00128 Roma (IT)
(74) Representative: Gitto, Serena
(86) International application number: PCT/IT2017/050008
(87) International publication number: WO 2018/122885

(56) References cited:
- WO-A1-2011/010337
- FRANCONI R ET AL: "Exploiting the plant secretory pathway to improve the anticancer activity of a plant-derived HPV16 E7 vaccine", INTERNATIONAL JOURNAL OF IMMUNOPATHOLOGY AND PHARMACOLOGY, SAGE PUBLICATIONS LTD, GB, vol. 19, no. 1, 1 January 2006 (2006-01-01), pages 187-197, XP009160218, ISSN: 0394-6320
- MASSA SILVIA ET AL: "Antitumor activity of DNA vaccines based on the human papillomavirus-16 E7 protein genetically fused to a plant virus coat protein", HUMAN GENE THE, MARY ANN LIEBERT, INC. PUBLISHERS, US, vol. 19, no. 4, 1 April 2008 (2008-04-01), pages 354-364, XP009129816, ISSN: 1043-0342, DOI: 10.1089/HUM.2007.122
- GIORGI C ET AL: "Human papillomavirus vaccines in plants", EXPERT REVIEW OF VACC, FUTURE DRUGS, LONDON, GB, vol. 9, no. 9, 1 January 2010 (2010-01-01) , pages 913-924, XP009181587, ISSN: 1476-0584, DOI: 10.1586/ERV.10.84
- YOSHIKATSU MATSUBAYASHI: "Posttranslationally Modified Small-Peptide Signals in Plants", ANNUAL REVIEW OF PLANT BIOLOGY, vol. 65, no. 1, 29 April 2014 (2014-04-29) , pages 385-413, XP055405271, US ISSN: 1543-5008, DOI: 10.1146/annurev-arplant-050312-120122
- EMANUELA NORIS ET AL: "A human papillomavirus 8 E7 protein produced in plants is able to trigger the mouse immune system and delay the development of skin lesions", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 156, no. 4, 15 January 2011 (2011-01-15), pages 587-595, XP019891267, ISSN: 1432-8798, DOI: 10.1007/S00705-010-0893-8
- VACULIK PETR ET AL: "Potato virus Xdisplaying the E7 peptide derived fromhuman papillomavirustype 16: a novel position for epitope presentation", PLANT CELL, TISSUE AND ORGAN CULTURE, SPRINGER, NL, vol. 120, no. 2, 2 October 2014 (2014-10-02), pages 671-680, XP035435921, ISSN: 0167-6857, DOI: 10.1007/S11240-014-0634-X [retrieved on 2014-10-02]
- SILVIA MASSA ET AL: "A plant protein signal sequence improved humoral immune response to HPV prophylactic and therapeutic DNA vaccines", HUMAN VACCINES AND IMMUNOTHERAPEUTICS, vol. 13, no. 2, 24 January 2017 (2017-01-24), pages 271-282, XP055451015, US ISSN: 2164-5515, DOI: 10.1080/21645515.2017.1264766
- CEROVSKA ET AL: "Transient expression of HPV16 E7 peptide (aa 44-60) and HPV16 L2 peptide (aa 108-120) on chimeric potyvirus-like particles using Potato virus X-based vector", PROTEIN EXPRESSION AND PURIFICATION, ACADEMIC PRESS, SAN DIEGO, CA, vol. 58, no. 1, 28 January 2008 (2008-01-28), pages 154-161, XP022435860, ISSN: 1046-5928, DOI: 10.1016/J.PEP.2007.09.006
- TOUBART ET AL: "Cloning and characterization of the gene encoding the endopolygalacturonase-inhibiting protein (PGIP) of Phaseolus vulgaris L", THE PLANT JOURNAL, BLACKWELL SCIENTIFIC PUBLICATIONS, OXFORD, GB, vol. 2, no. 3, 1 January 1992 (1992-01-01) , pages 367-373, XP002120515, ISSN: 0960-7412

## Description

The present invention concerns a DNA vaccine comprising the plant protein signal sequence as adjuvant as defined in the claims. The plant protein signal sequence drives the processing of an antigen through the mammalian secretion pathway and increases the efficacy of the DNA vaccine as defined in the claims for the use in the prevention and treatment of HPV etiology cancer, wherein the plant protein signal sequence is fused to the N-terminal portion of the antigen as defined in the claims.

DNA vaccines deliver genes encoding protein antigens into host cells, enabling their production to occur *in vivo* with many associated advantages: stimulation of both cellular and humoral immune response; possibility of manipulation to produce immunity against a specific target protein; modulation of the specificity and type of the immune response; rapid production; ease of production and storage.

The activation and strengthening of the immune response by means of innovative strategies is used in cancer therapy as well as in strengthening preventive vaccines for the resolution of infections, including emerging and re-emerging infections or those related to the onset of cancer (such as those related to infection with the human Papilloma Virus, HPV) which can have a significant medical impact on the population.

Preventive vaccines are formulations able to induce in the host an immune response, especially a humoral type immune response, which neutralizes the pathogen before it can develop the disease.

The latest generation of vaccines, which contains only parts of the pathogen which are known to be the target of immune responses, are obtained by exploiting advances in molecular immunology and genetic engineering.

An important goal in this area is to increase the efficiency of subunit vaccines through the identification of new adjuvants or increasing the immunogenic potential of antigens.

For a proper functioning of a prophylactic genetic vaccine it is necessary, in fact, that it provides adequate stimuli and humoral immune response.

However, at the present time of the research, one of the major problems to be solved with genetic vaccines (both prophylactic and therapeutic) is their poor ability to induce an adequate immune response (antibody production and cytotoxic /cell-mediated responses, respectively).

Numerous studies demonstrated the ability to increase the immune response obtainable with genetic vaccine by introducing genes coding for other molecules. For example, a method used for this aim was to insert in plasmids also genes which code for cytokines, molecules with signaling functions. In fact, the immune system cells release cytokines to adjust the mutual functioning, and some cytokines promote the activity of killer cells.

Recent studies indicate that a potent cellular response by killer T cells is critical to combat viral proliferation, as in the case of HIV.

Other studies have evaluated the possibility of including in the plasmids also the genes for chemokines, small molecules that attract antigen presenting cells and T cells to the damaged or infected tissues. Their specific mechanisms of action are still being studied. However, chemo/cytokines may induce detrimental secondary responses, tolerance or auto-immune reactions

In regards to cancers, Human Papillomavirus (HPV) is the agent behind the development of cervical squamous cell carcinoma, involved also in the pathogenesis of other cancers and in oropharyngeal carcinogenesis. HPVs have a causative role in 5% of all tumors globally, with a deep impact on public health for the number of cases and life-time medical costs due to specific treatment missing. Current strategies for treating HPV cancers have been only moderately successful and characterized by recurrence.¹ The available preventive vaccination (Gardasil, Gardasil-9 and Cervarix, based on the recombinant major capsid viral protein L1 of variously incident HPVs) will probably reduce the number of HPV cancer cases and of precancerous lesions worldwide, resulting in lives saved and related morbidity and cost-offsets.² Nevertheless, these benefits will be visible in the next decades and, without a profound shift in rates of vaccination in men and women worldwide, prophylactic vaccination will not have great impact at least on the incidence of certain HPV infections like those causing oropharyngeal cancers, that are currently in a dramatic rise.

In the context of HPV preventive vaccines, formulations based on the induction of broadly cross-neutralizing antibodies against L2, the minor HPV capsid protein, are being developed.³ Since L2 protein neutralization epitopes only become exposed via furin cleavage when L1 interacts with heparin sulfate-proteoglycans of the host, HPV infection produces predominantly L1 antibodies with little or no L2 antibodies. The alleviated selective pressure for antigenic variance leads to structural conservation of L2 that makes this a target for the development of a 'pan-HPV' vaccine.⁴ Neutralizing epitopes of L2 were found to map at the N-terminus of the protein (mainly spanning the first 88 aa)⁵ and, based on this evidence, recent strategies have employed heterologous, modified L2 oligopeptides created using repeats or grafted to various scaffold antigens (diphtheria toxin, flagellin etc.) or assembled in virus-like display vaccines to improve the cross-neutralization range and the antibody titers (for a review see ref. 4).

Due to the presence of viral E5-E6-E7 targets (tumor-associated antigens), HPV tumors are also highly amenable to immunotherapeutic approaches, with the possibility to set specific treatments based on vaccination eventually used in parallel with chemo-/radio-therapy and other currently available non-specific treatments, to integrate primary and secondary prevention. In this field, a strategy able to trigger E5/E6/E7-specific CTLs destroying HPV-infected and/or transformed cells is desirable but challenging, since HPV tumor-associated antigens are typically weak antigens with low immunogenicity (like many tumor antigens), active in virus immune evasion, and unable to induce inflammation.

As regards cancers associated with HPV infection, the genetic vaccine VGX-3100, a mixture of two plasmids that encode the optimized consensus E6 and E7 genes of HPV serotypes 16 and 18, respectively, delivered by electroporation, is the first therapeutic vaccine able to induce histopathological regression in women with CIN2/3associated with HPV-16 and HPV-18 (clinical trial phase 2b).⁶

In the context of DNA therapeutic vaccination, a previous strategy of the inventors was to fuse an attenuated HPV16 E7 gene (E7GGG also named E7* in the present description, for example in the figures) to the N-Terminus of the coat protein (CP) sequence of Potato virus X (PVX), a carrier capable of generating CD4+ T cell responses that may enhance CD8+ T cell priming through the presentation within MHC class II pathway. This fusion construct was able to inhibit the growth of a HPV-expressing experimental mouse tumor.⁷

In addition, Cerovska et al., 2008⁸ disclosed the transient expression of HPV16 E7 peptide (aa 44-60) and HPV16 L2 peptide (aa 108-120) on chimeric potyvirus-like particles using Potato virus X-based vector.

In the light of above it is therefore apparent the need to provide for new DNA vaccines able to overcome the disadvantages of the known preventive and therapeutic DNA vaccines.

It is known that the signal sequence (ss) derived from the polygalacturonase-inhibiting protein (PGIP) from *Phaseolus vulgaris* increases HPV16 E7 protein production yield in plants since it is able to drive the HPV16 E7 protein in the plant secretory compartment. The protein was expressed with a 5-fold change accumulation level compared to the cytoplasmic, unfused version of the vaccine by transient plant expression. The possibility to increase the quantity of the E7 protein administered per dose, led to a higher cell-mediated immunogenicity specific to E7 of the vaccine and, ultimately, the growth inhibition of E7-expressing experimental tumours was achieved.⁹

According to the present invention, it is now found that the signal sequence of PGIP (PGIPss) is able to exert a biological activity when delivered into mammalian cells by transfection, eliciting a strong antibody response when it is fused to an antigen, said response being higher than that elicited by the antigen per se. Therefore, it is shown for the first time that a plant signal sequence is able to perform its function in a mammalian cell line. In particular, it was demonstrated that the signal sequence of PGIP can be advantageously used as adjuvant in preventive (HPV) DNA vaccines or preventing/therapeutic DNA vaccines. Initially, the potential effect of the PGIPss in an expression vector for mammalian cells on the intracellular trafficking and export of HPV antigens was evaluated. DNA plasmids encoding the plant ss preceding a HPV16 E7 mutated sequence (E7GGG) fused or not to the coat protein of PVX (CP) carrier were constructed. The ss demonstrated to induce the intracellular re-positioning of the E7GGG protein from a nuclear to a cytoplasmic location and an E7-specific, strong humoral response in animal models.

Subsequently, PGIPss was genetically fused to the cross-reactive epitope of the L2 protein of HPV16 (L2₁₋₂₀₀, aa 1-200) and to the chimeric L2-E7GGG gene to obtain a therapeutic/preventive pan-reactive DNA vaccine.

Therefore, DNA vaccines against HPV both with preventive value (L2 antigen of the viral capsid) and preventive / therapeutic value (L2-E7GGG fusion antigen mutated early in the oncogenic activity sites) were constructed. In fact, anti-L2 antibodies block the infection and the entry of the virus into the cell whereas cytotoxic lymphocytes (CTL) against the epitopes of E7, which are expressed by the infected / transformed cell, are able to cure the infection and the tumor to it related.

As previously anticipated, upon immunization with DNA plasmids harbouring PGIPss preceding the E7GGG antigen fused or not to the PVX CP gene, the translated signal peptide directed the nascent E7GGG protein into the endoplasmic reticulum and Golgi pathway of the transfected host cells, facilitating secretion of E7GGG. In fact, the expression of the proteins was characterized *in vitro* by transfection of HEK-293 cells and the E7GGG protein was found in culture media of transfected cells, suggesting that it was targeted in the secretory pathway by the signal peptide that, ultimately, determined its secretion. In effect, immunofluorescence data showed that PGIPss shifts E7GGG from a mainly nuclear to a cytoplasmic location. On the other hand, the lysates of the transfected cells showed that the produced fusion antigens separated in a double band in immunoblotting, probably because PGIPss is not always properly recognized. However, this is the first demonstration that a signal sequence of a plant protein is able to exert a biological activity in mammalian cells.

The effectiveness of these secretory DNA vaccines in inducing humoral and cell-mediated responses was studied. In general, DNA vaccines are considered strong inducers of Th1 responses. In the current study, it was observed that the secretory constructs elicited both humoral and cell-mediated immunity even if the latter response was weaker with respect to the non-secretory counterparts used as control, as shown by IFN-γ production in re-stimulated spleen cells from vaccinated mice. This poor response affected outcomes of tumor challenged mice because induction of a strong cell-mediated immunity is strictly linked to protection against HPV tumors. Indeed, animals that did not develop tumors after challenge, showed higher levels of IFN-γ secreting splenocytes (data not shown). On the other hand, although not the crucial one for HPV tumor resolution, antibody response was astonishingly high compared to that of non-secretory constructs.

Thus, this activity of the plant ss was utilized for designing a DNA 'pan'-reactive therapeutic/preventive HPV vaccine. The plant ss was genetically fused to a cross-reactive epitope of the L2 protein from HPV16 (L2₁₋₂₀₀) and fusions were made also with the E7GGG sequence. The L2 peptide was successfully expressed in *E*. *coli* to set up ELISA experiments and protein expression in mammalian cells was demonstrated, confirming that the plant protein signal is active in mammalian cells.

One of the issues regarding DNA vaccine efficiency is the way of its delivery. Conventional injection of DNA is considered a key limitation due to inefficient uptake by cells. Electroporation (EP) appears a promising approach for improving immunogenicity of DNA vaccines for its ability to increase cellular permeability resulting in a high level of protein expression and improved immune response.¹⁰ Recently, clinical trials have showed the efficiency of EP in humans.^{11,12} According to the present invention, the data confirmed that EP in more efficient than IM injection. Indeed, quite disappointing results were obtained after using a conventional IM vaccination schedule (data not shown), whereas a short vaccination program by EP and ID injection¹⁰ gave rise to strong humoral response by PGIPss-linked constructs. Moreover, EP was able to induce a longstanding humoral immune response against L2₁₋₂₀₀, at least persisting six months in the utilized mouse model.

In conclusion, the experimental results from this study demonstrate for the first time that a plant signal sequence can exert an activity of targeting to secretion also in mammalian cells. According to the present invention, it has been identified that the ss of PGIP is an optimal signal peptide for E7GGG secretion. Moreover, this ss plays a critical role in inducing a strong humoral response against E7GGG and L2₁₋₂₀₀ antigens of HPV16. In addition, ss-constructs delivered by EP are able to induce a rapid, robust, and durable humoral response. This result is important because DNA vaccines suffer from poor IgG induction and were believed not efficacious as prophylactic vaccines. Preliminary experiments seem to indicate the neutralizing nature of the anti-L2 antibodies (data not shown), making this a very important step in the field of HPV preventive/therapeutic vaccination against HPV.

The present invention highlights also the important interplay between a plant signal peptide and mammalian intracellular trafficking that may lead to the induction of humoral response upon immunization. One explanation for this surprising result can be found in the structure and function of ss. The secretion function has been conserved from bacteria through eukaryotes and, though diverse in length/composition, ss share a conserved tripartite structure: 20-30 residues in length with a basic "N domain", a 7-13 residue hydrophobic "H domain" and a slightly polar "C domain".¹³ In addition to this common structure, conservation of residues in particular positions of the mammalian signal peptide sequences is emerging. In this sense, the present results are corroborated by very recent findings showing that in mammalian ss, small neutral amino acids are prevalent in -1 (last amino acid of the signal peptide) and -3 positions, whereas bulky aromatic amino acids are often found at -2 position and proline residues are virtually absent from all positions from -3 to +1 (first amino acid of the mature peptide) positions.¹⁴ Interestingly, these features of mammalian ss are found also in PGIP ss where -1 position corresponds to serine and -3 position to alanine and position -2 corresponds indeed to leucine. Moreover, proline residues are absent from all positions from -3 to +1 both in the fusion with E7GGG and with L2₁₋₂₀₀.

It is therefore plausible that other plant signal sequences fused to other antigens are effective in eliciting strong immune responses in mammalian cells, particularly, the plant signal sequences and antigens which have the amino acidic characteristics described above.

The potential of using heterologous signal peptides to target proteins might be significant to many areas of biological research as well as the biopharmaceutical industry. In particular, since the ability to express a protein of interest of a certain quality upon DNA immunization is critical for building reliable vaccinogens able to trigger an adequate immune response, the possibility to use signal sequences of plant origin to drive antigen trafficking in different compartments of mammalian cells offers new perspectives in the design of new preventive and therapeutic vaccines, such as HPV vaccines (chimeric ss-L2₁₋₂₀₀-E7GGG vaccine). Beyond the effects observed on the immunity triggered to the above-mentioned antigens, plant ss such as PGIPss can be used in the rational design of genetic vaccines against other tumor-associated pathogens and against emerging and re-emerging infectious agents.

The advantages of the present invention are:
- obtaining a high antibody response against an antigen encoded in the DNA vaccine as defined in the claims;
- providing the short nucleotide sequence encoding the plant protein signal sequence as defined in the claims into a single plasmid and that stimulates a strong antibody response;
- providing a vaccine against HPV etiology cancer using a single plasmid structure in which to insert the antigenic sequence of fusion and which can be easily produced on an industrial scale according to GMP;
- possibility of using antigenic structures also relatively large, since the fusion concerns the addition of a few nucleotides;
- providing a more effective and safe vaccine because it does not involve the production of chemo/cytokines which may induce secondary responses, or of animal antigens that could cause cross autoimmune responses.

It is therefore a specific object of the present invention to provide a DNA (or genetic) vaccine comprising a polynucleotide sequence which encodes a fusion protein, or an expression vector or plasmid comprising said polynucleotide sequence, said fusion protein being SEQ ID NO:32.

The plant protein signal sequence according to the present invention is the plant signal sequence of polygalacturonase-inhibiting protein 1 (PGIP1ss) of *Phaseolus vulgaris*¹⁵*.*

The DNA vaccine according to the present invention can be used for different immunological purposes (plurivalent vaccination or preventive/therapeutic vaccination). Therefore, the plant protein signal sequence according to present invention can be advantageously used as adjuvant in DNA vaccine prevention and therapy.

The antigen contains mutations in order to make it safer. For instance E7 of HPV16 is mutated in the binding region of the protein of Retinoblastoma in order to make it allegedly non oncogenic (the attenuated E7GGG is described in Massa et al. 2008)⁷.

According to an embodiment of the present invention, DNA vaccine is a pharmaceutical composition which further comprises one or more excipients and/or adjuvants.

The DNA vaccine according to the present invention can be administered by electroporation.

The present invention concerns also the DNA vaccine as defined above for use in the prevention and treatment of HPV etiology cancer.

As mentioned above, the DNA vaccine can be administered by electroporation.

It is a further object of the present invention, a process for the preparation of the DNA vaccine as defined above, said process comprising a) preparing a fusion gene encoding the fusion protein of sequence SEQ ID NO:32 by fusing a nucleotide sequence encoding the plant protein signal sequence of polygalacturonase-inhibiting protein, said plant protein signal sequence having sequence MTQFNIPVTMSSSLSIILVILVSLRTALS (SEQ ID NO:1), to the N-terminal portion of a nucleotide sequence encoding an antigenic sequence consisting of the immunogenic fragment consisting of aminoacids 1-200 of L2 of HPV16 and E7GGG of HPV16; b) cloning the fusion gene in a plasmid vector for expression in mammalian cells, such as pcDNA3.1, or pVAX; and c) amplification of the plasmid vector in a suitable bacterial microorganism, such as *E*. *coli,* and isolation thereof from the microorganism.

The DNA or genetic vaccine according to the present invention can be both prophylactic and therapeutic vaccine and can be used for treating both animals and human beings i.e. it can be used in both medical and veterinary fields.

The present invention now will be described by an illustrative, but not limitative way, according to preferred embodiments thereof, with particular reference to enclosed drawings, wherein:

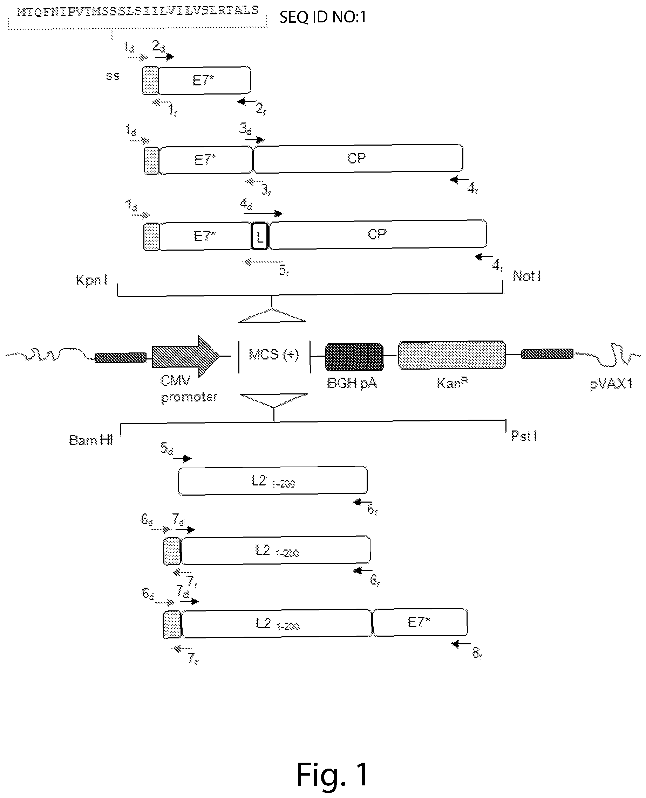

**Figure 1** shows a schematic representation of the genes introduced into the mammalian vector pVAX1 with indication of the oligonucleotides used for PCR amplification and assembly (see Table 1). The recombinant genes are under the control of the CMV promoter and the BGH pA signal constituting the expression cassette, while the selectable marker is KanR. BGH pA: Bovine Growth Hormone poly-adenilation signal; KanR: Kanamycin Resistance; *ss* (light gray boxes): signal sequence of the gene encoding the Polygalacturonase Inhibiting Protein (PGIP) of *Phaseolus vulgaris*; E7*: attenuated E7 gene of HPV16, E7GGG; CP: Potato Virus X coat protein gene; L: linker sequence encoding the Gly-Pro-Gly-Pro tetrapeptide (SEQ ID NO:25); L2₁₋₂₀₀: nucleotide sequence corresponding to aa 1-200 of the L2 minor capsid protein of HPV type 16.

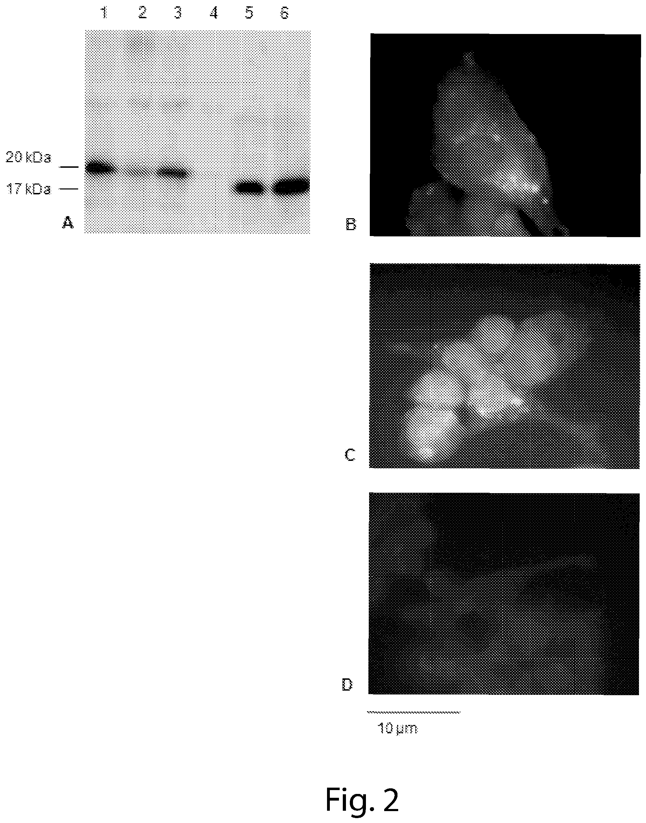

**Figure 2** shows the expression of the E7GGG protein in HEK-293 cells upon transfection. A: Western blot of total soluble proteins from 1×10⁶ cells transfected with E7GGG +/- ss and of culture media. 1) ss-E7GGG (cell lysate); 2) ss-E7GGG culture medium; 3) E7GGG (cell lysate); 4) E7GGG culture medium; 5), 6) purified His₆-E7GGG from *E*. *coli* 25 ng and 50 ng, respectively. The E7GGG protein runs as a 20 kDa protein in HEK-293 cells, while His₆-E7* shows a slightly lower molecular weight in bacteria. B, C, D: Immunofluorescence (FITC) detected in cells transiently transfected with ss-E7GGG (b), with E7GGG (c) and non-transfected HEK-293 cells used as a negative control (d) (original magnification, 100x; scale bar, 10 µm).

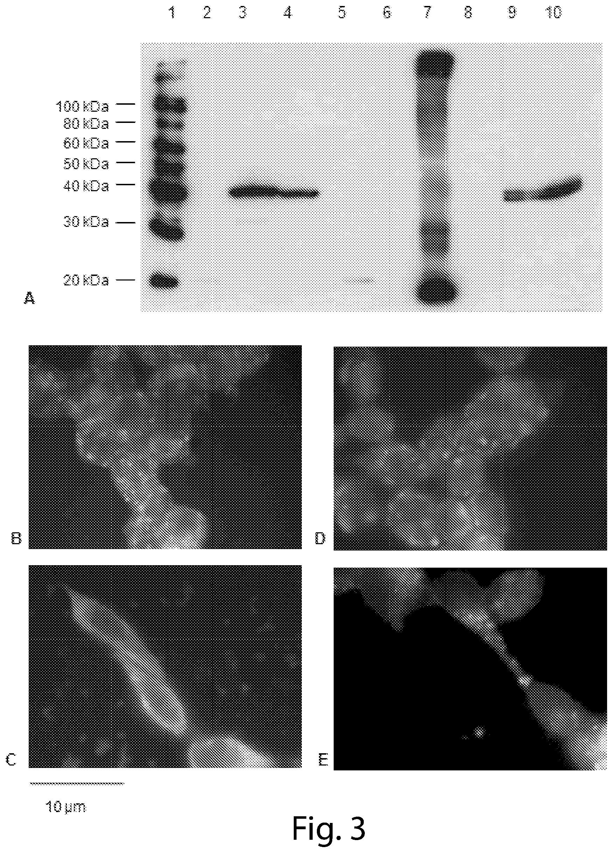

**Figure 3** shows the expression of the E7GGG-CP proteins in HEK-293 cells upon transfection. A: Immunoblotting of proteins from lysates of 1×10⁶ cells transfected with the E7GGG-CP fusions with or without ss. 1) Biotinylated Protein Ladder NEB; 2) E7GGG (cell lysate); 3) E7GGG-CP (cell lysate); 4) E7GGG-L-CP (cell lysate); 5) ss-E7GGG (cell lysate); 6) ss-E7*-CP (culture medium); 7) ss-E7 plant extract; 8) ss-E7GGG-L-CP (culture medium); 9) ss-E7GGG-CP (cell lysate); 10) ss-E7GGG -L-CP (cell lysate). B, C, D, E: Immunofluorescence (FITC) detected in cells transfected with E7GGG-CP (B, C) and E7GGG -L-CP (D, E) plasmids without or with the ss, respectively (original magnification, 100x; scale bar, 10 µm).

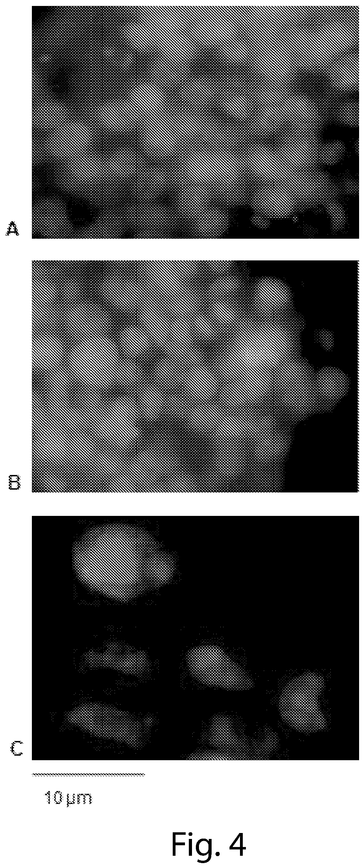

**Figure 4** shows the expression of the L2₁₋₂₀₀ proteins in HEK-293 cells upon transfection. Immunofluorescence in cells transfected with L2₁₋₂₀₀ (A), ss-L2₁₋₂₀₀ (B) and ss-L2₁₋₂₀₀-E7GGG (C) constructs. FITC fluorescence was coupled with DAPI nuclei blue counter-staining (original magnification, 100x; scale bar, 10 µm).

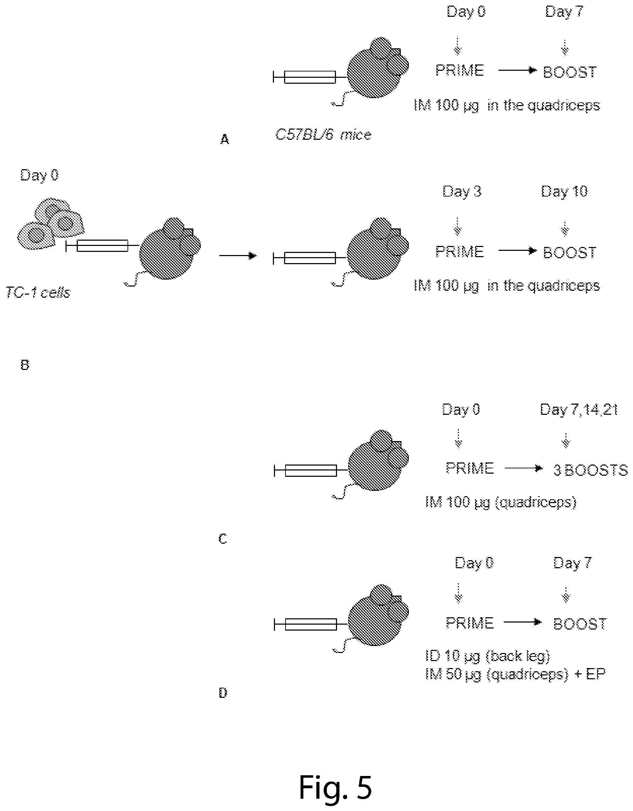

**Figure 5** shows the immunization schedules. Immunization with the ss-E7GGG +/-CP series in absence (A) or in presence of tumor challenge (B) to evaluate immune response and tumor rejection, respectively. Long-term (C) and short-term (D) immunization protocols used to induce antibody response in mice vaccinated with the L2₁₋₂₀₀ +/- E7GGG series. ID: Intra-dermal injection; IM: Intra-muscular injection; EP: Electroporation.

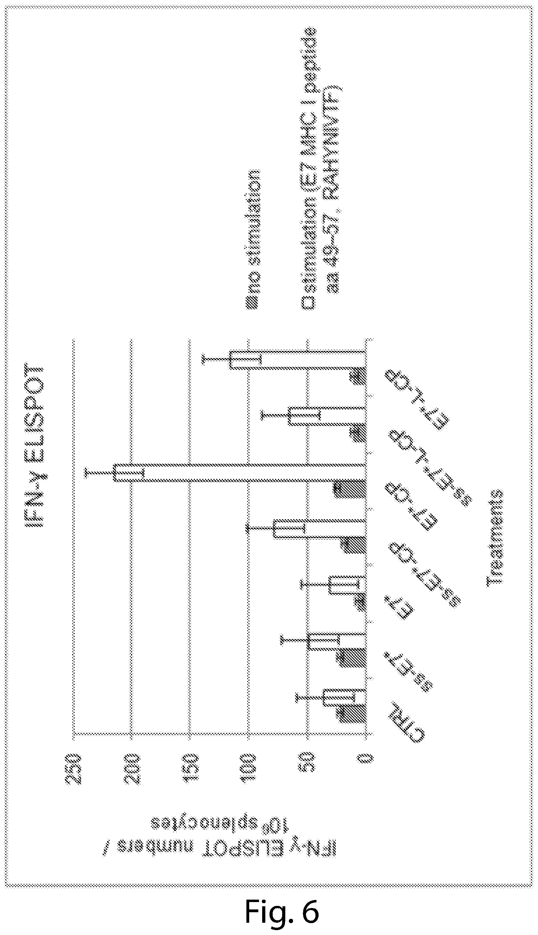

**Figure 6** shows the results of ELISPOT assay for IFN-γ-secreting splenocytes after vaccination with ss-E7GGG +/- CP in C57BL/6 mice. Data are presented as mean spot number ± SD per 1 × 10⁶ splenocytes from triplicate wells. Cells were stimulated with an E7 specific H-2Db cytotoxic T lymphocyte MHC class I epitope. Gray columns represent non-stimulated splenocytes. The presence of IFN-γ producing E7 specific T-cell precursors was determined using an anti-IFN-γ antibody, as described in Material and Methods.

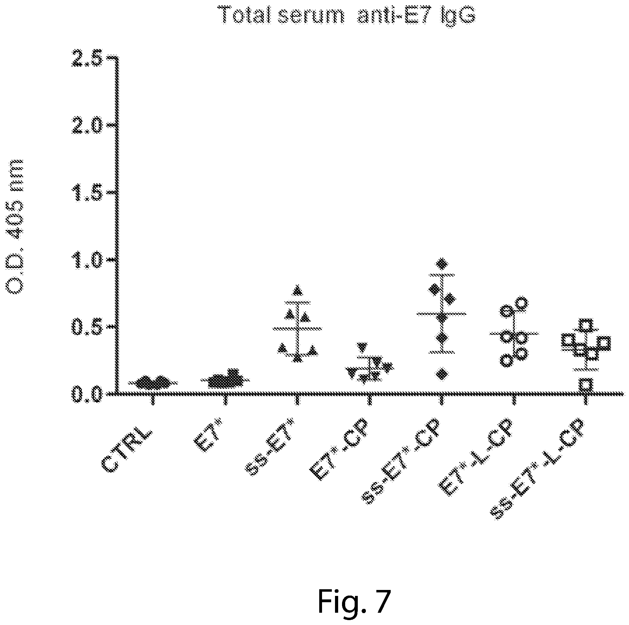

**Figure 7** shows the humoral immunological response in mice after vaccination with ss-E7GGG +/- CP. Data for ELISA test are reported from a representative experiment. Humoral E7-specific serum IgG responses are presented as optical density values at 405 nm of 1:100 diluted sera. The mean titer value from each group is marked with a dash. Means and variances are significantly different (p< 0,05) according to the One-way ANOVA test and to the Bonferroni's multiple comparison test.

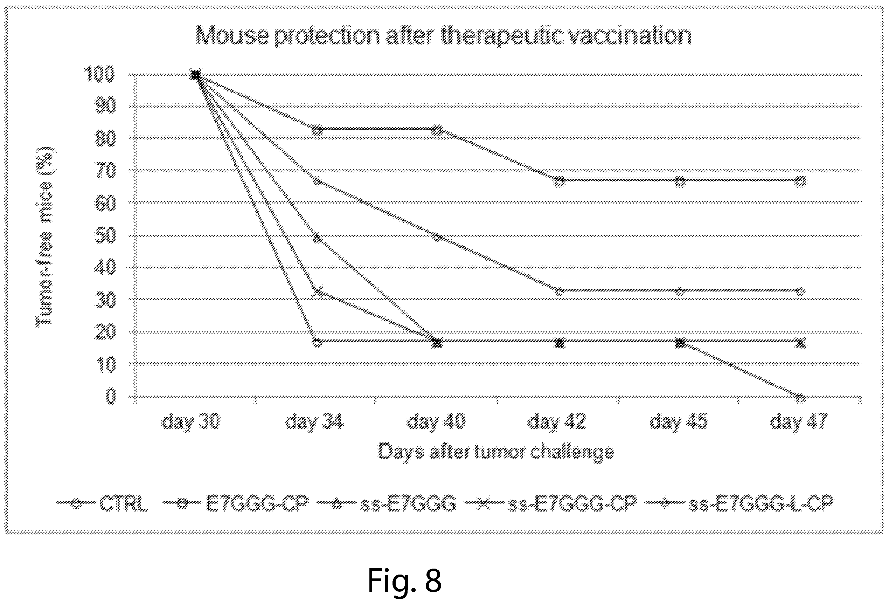

**Figure 8** shows the growth inhibition of experimental TC-1-induced tumors after therapeutic vaccination of mice with ss-E7GGG, ss-E7GGG - CP and ss-E7GGG -L-CP. Results are presented as the percentage of tumor-free mice. The presence of tumors was monitored by palpation twice per week. The animals were sacrificed on day 47 after tumor challenge, when all the animals with tumors were euthanized for ethical reasons. E7GGG -CP was administered for comparison of efficacy data.

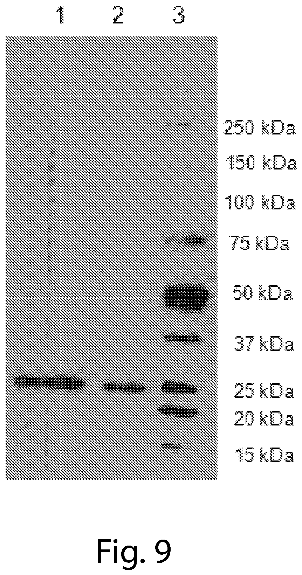

**Figure 9** shows L2₁₋₂₀₀ expression in *E*. *coli.* Lane 1: L2₁₋₂₀₀ purified under native conditions; lane 2: L2₁₋₂₀₀ purified under denaturing conditions; lane 3 Molecular weight Marker Precision Plus Protein^{™} Dual Color Standards (Bio-Rad Milan, Italy).

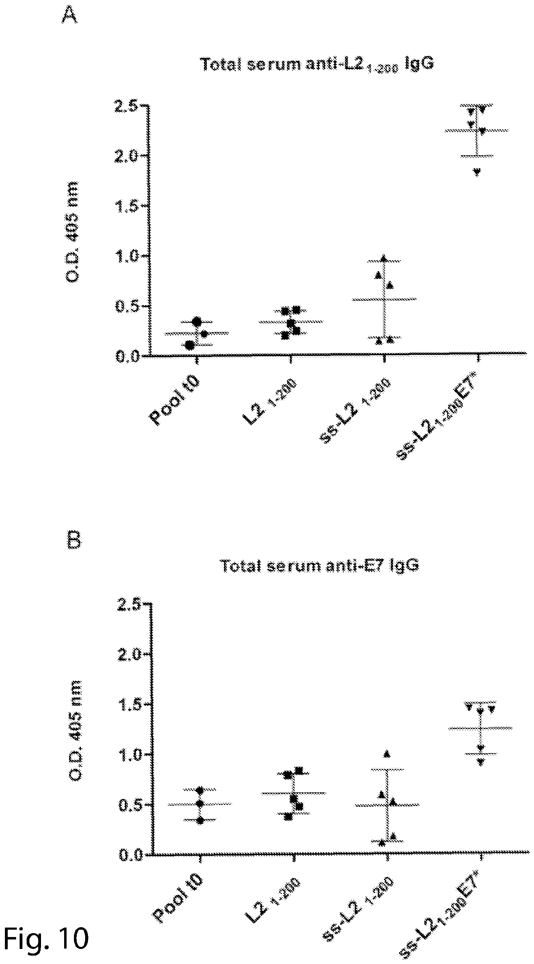

**Figure 10** shows humoral immunological response to L2₁₋₂₀₀ and E7GGG in mice after vaccination with the L2₁₋₂₀₀, ss-L2₁₋₂₀₀ and ss-L2₁₋₂₀₀-E7GGG. C57BL/6 mice were immunized as described in figure 5D. Data for ELISA test were generated for sera collected from every mouse (m 1-5: mouse 1-5) of each vaccination group after coating with either His₆-L2₁₋₂₀₀ (Fig. 10A) or His₆-E7 (Fig. 10B) and are reported from one representative experiment. Humoral specific serum IgG responses are presented as optical density values at 405 nm of 1:100 diluted sera. The mean titer value from each group is marked with a dash. Pool t0: average of pool of pre immune sera for each treatments. Means and variances are significantly different (p< 0,05) according to the One-way ANOVA test and to the Bonferroni's multiple comparison test.

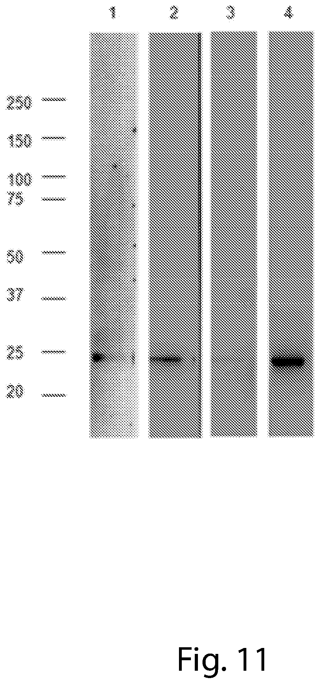

**Figure 11** shows reactivity of sera of immunized mice against the L2₁₋₂₀₀ peptide 6 months post-immunization, as described in figure 5D. 50ng of L2₁₋₂₀₀ were loaded for each lane and revealed with RG-1 monoclonal antibody (strip 1) or 1:100 dilutions of pooled sera from ss-L2₁₋₂₀₀ (strip 2), L2₁₋₂₀₀ (strip 3), and ss-L2₁₋₂₀₀-E7GGG (strip 4) mouse groups.

### EXAMPLE 1: Preparation and study of DNA vaccines according to the present invention

### Material & Methods

### Plasmids

All utilized primers are indicated in figure 1 and listed in table 1 (note that all upstream primers had a Kozak sequence for expression regulation in mammals).

**Table 1. DNA primer list with sequences, templates and PCR products.**

| primer id. | sequence 5' - 3' | template | PCR and assembly product |
|---|---|---|---|
| 1d | G GCC GGT ACC ATC ATG ACT CAA TTC AAT ATC CAA G (SEQ ID NO: 2) | PGIP ss | ss-E7* |
| | KpnI site | | |
| 1r | TGT AGG TGT ATC TCC ATG CAT TGA GAG TGC AGT TCT CAA (SEQ ID NO: 3) | | |
| | upstream nucleotides of E7* + downstream nucleotides of ss | | |
| 2d | TTG AGA ACT GCA CTC TCA ATG CAT GGA GAT ACA CCT ACA (SEQ ID NO: 4) downstream nucleotides of ss + | E7* | |
| | upstream nucleotides of E7* | | |
| 2r | TTC TCG ACT TGC GGC CGC TTA TGG TTT CTG AGA ACA GAT (SEQ ID NO: 5) | | |
| | NotI site | | |
| 1d | G GCC GGT ACC ATC ATG ACT CAA TTC AAT ATC CAA G (SEQ ID NO: 6) | ss-E7* | ss-E7*-CP |
| | (see above) | | |
| 3r | CTG TGT TGT GCT AGC TGG TGG TTT CTG AGA ACA GAT GGG (SEQ ID NO: 7) | | |
| | upstream nucleotides of CP + downstream nucleotides of E7* | | |
| 3d | ATC TGT TCT CAG AAA CCA CCA GCT AGC ACA ACA CAG CCC (SEQ ID NO: 8) downstream nucleotides of E7* + | CP | |
| | upstream nucleotides of CP | | |
| 4r | TTC TCG ACT TGC GGC CGC TTA TGG TGG TGG TAG AGT GAC (SEQ ID NO: 9) | | |
| | NotI site | | |
| 1d | G GCC GGT ACC ATC ATG ACT CAA TTC AAT ATC CAA G (SEQ ID NO: 10) | ss-E7* | ss-E7*-L-CP |
| | (see above) | | |
| 5r | GCT AGC TGG AGG TCC GGG TCC TGG TTT CTG AGA ACA GAT GGG (SEQ ID NO: 11) | | |
| | upstream nucleotides of CP + linker sequence + downstream nucleotides of E7* | | |
| 4d | CAG AAA CCA GGA CCC GGA CCT CCA GCT AGC ACA ACA CAG CCC (SEQ ID NO: 12) | CP | |
| | downstream nucleotides of E7*+ linker sequence + upstream nucleotides of CP | | |
| 4r | TTC TCG ACT TGC GGC CGC TTA TGG TGG TGG TAG AGT GAC (SEQ ID NO: 13) | | |
| | (see above) | | |
| 5d | GGCCGGATCC**ATC**ATGCGTCACAAACGTTCAG (SEQ ID NO: 14) | synthetic L2₁₋₂₀₀-E7* | L2₁₋₂₀₀ |
| | BamHI site | | |
| 6r | GGCCCTGCAGTTAAAATGTATCCATCGGAATT (SEQ ID NO: 15) | | |
| | PstI site | | |
| 6d | GGCCGGATCC**ATC**ATGACTCAATTCAATATCCAAG(SEQ ID NO: 16) | PGIP ss | ss- L2₁₋₂₀₀ |
| | BamHI site | | |
| 7r | TGAACGTTTGTGGACGCATTGAGAGTGCAGTTCTCAA (SEQ ID NO: 17) | | |
| | upstream nucleotides of L2₁₋₂₀₀ + downstream nucleotides of ss | | |
| 7d | TTGAGAACTGCACTCTCAATGCGTCACAAACGTTCA (SEQ ID NO: 18) | synthetic L2₁₋₂₀₀-E7* | |
| | downstream nucleotides of ss + upstream nucleotides of HPV16 L2 | | |
| 6r | GGCCCTGCAGTTAAAATGTATCCATCGGAATT (SEQ ID NO: 19) (see above) | | |
| 6d | GGCCGGATCC**ATC**ATGACTCAATTCAATATCCAAG(SEQ ID NO: 20) | PGIP ss | ss- L2₁₋₂₀₀-E7* |
| | (see above) | | |
| 7r | TGAACGTTTGTGGACGCATTGAGAGTGCAGTTCTCAA (SEQ ID NO: 21) | | |
| | (see above) | | |
| 7d | TTGAGAACTGCACTCTCAATGCGTCACAAACGTTCA (SEQ ID NO: 22) | synthetic L2₁₋₂₀₀-E7* | |
| | (see above) | | |
| 8r | GGCCCTGCAGTTATGGTTTCTGAGAACAGATG (SEQ ID NO: 23) | | |
| | PstI site | | |

*Primer identification (id.) is as shown in* *figure 1**; restriction sites are underlined and Kozak sequence is in bold (d: direct, r: reverse).*

The protein sequences used in the present study are listed below:
**SEQ ID NO:24** - E7-specific H-2Db cytotoxic T lymphocyte (CTL) MHC class I epitope (amino acids 49-57)
   RAHYNIVTF
**SEQ ID NO:25** - **L linker**
   GPGP
**SEQ ID NO:26** - **E7 (HPV16 E7 protein)** 
1 M H G D T P T L H E Y M L D L Q P E T T
21 D L Y C Y E Q L N D S S E E E D E I D G
41 P A G Q A E P D R A H Y N I V T F C C K
61 C D S T L R L C V Q S T H V D I R T L E
81 D L L M G T L G I V C P I C S Q K P *
**SEQ ID NO: 27 - E7* or also named as E7GGG protein from HPV16 E7 mutagenesis** 
1 M H G D T P T L H E Y M L D L Q P E T T
21 G L Y G Y G Q L N D S S E E E D E I D G
41 P A G Q A E P D R A H Y N I V T F C C K
61 C D S T L R L C V Q S T H V D I R T L E
81 D L L M G T L G I V C P I C S Q K P *
**SEQ ID NO: 28 - L2 (1-200) fragment protein i.e. HPV16 L2₁₋₂₀₀ which is optimized for the codon usage of *Chlamydomonas rehinardtii*** 
1 M R H K R S A K R T K R A S A T Q L Y K
21 T C K Q A G T C P P D I I P K V E G K T
41 I A E Q I L Q Y G S M G V F F G G L G I
61 G T G S G T G G R T G Y I P L G T R P P
81 T A T D T L A P V R P P L T V D P V G P
 
101 S D P S I V S L V E E T S F I D A G A P
121 T S V P S I P P D V S G F S I T T S T D
141 T T P A I L D I N N T V T T V T T H N N
161 P T F T D P S V L Q P P T P A E T G G H
181 F T L S S S T I S T H N Y E E I P M D T
201 F *
**SEQ ID NO: 29** - **CP protein** 
1 M P A S T T Q P I G S T T S T T T K T A
21 G A T P A T A S G L F T I P D G D F F S
41 T A R A I V A S N A V A T N E D L S K I
61 E A I W K D M K V P T D T M A Q A A W D
81 L V R H C A D V G S S A Q T E M I D T G
101 P Y S N G I S R A R L A A A I K E V C T
121 L R Q F C M K Y A P V V W N W M L T N N
141 S P P A N W Q A Q G F K P E H K F A A F
161 D F F N G V T N P A A I M P K E G L I R
181 P P S E A E M N A A Q T A A F V K I T K
201 A R A Q S N D F A S L D A A V T R G R I
221 T G T T T A E A V V T L P P P *
**SEQ ID NO: 30 - ss-E7*-CP = ssE7GGG-CP** 
1 M T Q F N I P V T M S S S L S I I L V I

21 L V S L R T A L S H G D T P T L H E Y M

41 L D L Q P E T T G L Y G Y G Q L N D S S

61 E E E D E I D G P A G Q A E P D R A H Y
 
81 N I V T F C C K C D S T L R L C V Q S T
101 H V D I R T L E D L L M G T L G I V C P
121 I C S Q K P P A S T T Q P I G S T T S T
141 T T K T A G A T P A T A S G L F T I P D
161 G D F F S T A R A I V A S N A V A T N E
181 D L S K I E A I W K D M K V P T D T M A
201 Q A A W D L V R H C A D V G S S A Q T E
221 M I D T G P Y S N G I S R A R L A A A I
241 K E V C T L R Q F C M K Y A P V V W N W
261 M L T N N S P P A N W Q A Q G F K P E H
281 K F A A F D F F N G V T N P A A I M P K
301 E G L I R P P S E A E M N A A Q T A A F
321 V K I T K A R A Q S N D F A S L D A A V
341 T R G R I T G T T T A E A V V T L P P P
361 *
**SEQ ID NO: 31 - ss-E7*-L-CP= ssE7GGG-L-CP** 
1 M T Q F N I P V T M S S S L S I I L V I
21 L V S L R T A L S M H G D T P T L H E Y
41 M L D L Q P E T T G L Y G Y G Q L N D S
61 S E E E D E I D G P A G Q A E P D R A H
81 Y N I V T F C C K C D S T L R L C V Q S
101 T H V D I R T L E D L L M G T L G I V C
121 P I C S Q K P G P G P P A S T T Q P I G
141 S T T S T T T K T A G A T P A T A S G L
161 F T I P D G D F F S T A R A I V A S N A
 
181 V A T N E D L S K I E A I W K D M K V P

201 T D T M A Q A A W D L V R H C A D V G S

221 S A Q T E M I D T G P Y S N G I S R A R

241 L A A A I K E V C T L R Q F C M K Y A P

261 V V W N W M L T N N S P P A N W Q A Q G

281 F K P E H K F A A F D F F N G V T N P A

301 A I M P K E G L I R P P S E A E M N A A

321 Q T A A F V K I T K A R A Q S N D F A S

341 L D A A V T R G R I T G T T T A E A V V

361 T L P P P *
**SEQ ID NO: 32 - ss-L2(1-200)-E7* = ss-L2(1-200)-E7GGG** 
1 M T Q F N I P V T M S S S L S I I L V I
21 L V S L R T A L S R H K R S A K R T K R
41 A S A T Q L Y K T C K Q A G T C P P D I
61 I P K V E G K T I A E Q I L Q Y G S M G
81 V F F G G L G I G T G S G T G G R T G Y
101 I P L G T R P P T A T D T L A P V R P P
121 L T V D P V G P S D P S I V S L V E E T
141 S F I D A G A P T S V P S I P P D V S G
161 F S I T T S T D T T P A I L D I N N T V
181 T T V T T H N N P T F T D P S V L Q P P
201 T P A E T G G H F T L S S S T I S T H N
221 Y E E I P M D T F H G D T P T L H E Y M
241 L D L Q P E T T G L Y G Y G Q L N D S S
261 E E E D E I D G P A G Q A E P D R A H Y
 
281 N I V T F C C K C D S T L R L C V Q S T

301 H V D I R T L E D L L M G T L G I V C P

321 I C S Q K P *
**SEQ ID NO: 33 - E7*-CP = E7GGG-CP** 
1 M H G D T P T L H E Y M L D L Q P E T T

21 G L Y G Y G Q L N D S S E E E D E I D G

41 P A G Q A E P D R A H Y N I V T F C C K

61 C D S T L R L C V Q S T H V D I R T L E

81 D L L M G T L G I V C P I C S Q K P P A

101 S T T Q P I G S T T S T T T K T A G A T

121 P A T A S G L F T I P D G D F F S T A R

141 A I V A S N A V A T N E D L S K I E A I

161 W K D M K V P T D T M A Q A A W D L V R

181 H C A D V G S S A Q T E M I D T G P Y S

201 N G I S R A R L A A A I K E V C T L R Q

221 F C M K Y A P V V W N W M L T N N S P P

241 A N W Q A Q G F K P E H K F A A F D F F

261 N G V T N P A A I M P K E G L I R P P S

281 E A E M N A A Q T A A F V K I T K A R A

301 Q S N D F A S L D A A V T R G R I T G T

321 T T A E A V V T L P P P *
**SEQ ID NO: 34** - **E7*-L-CP= E7GGG-L-CP** 
1 M H G D T P T L H E Y M L D L Q P E T T

21 G L Y G Y G Q L N D S S E E E D E I D G
 
41 P A G Q A E P D R A H Y N I V T F C C K

61 C D S T L R L C V Q S T H V D I R T L E

81 D L L M G T L G I V C P I C S Q K P G P

101 G P P A S T T Q P I G S T T S T T T K T

121 A G A T P A T A S G L F T I P D G D F F

141 S T A R A I V A S N A V A T N E D L S K

161 I E A I W K D M K V P T D T M A Q A A W

181 D L V R H C A D V G S S A Q T E M I D T

201 G P Y S N G I S R A R L A A A I K E V C

221 T L R Q F C M K Y A P V V W N W M L T N

241 N S P P A N W Q A Q G F K P E H K F A A

261 F D F F N G V T N P A A I M P K E G L I

281 R P P S E A E M N A A Q T A A F V K I T

301 K A R A Q S N D F A S L D A A V T R G R

321 I T G T T T A E A V V T L P P P *
**SEQ ID NO: 35** - **ssE7GGG** 
1 M T Q F N I P V T M S S S L S I I L V I
21 L V S L R T A L S M H G D T P T L H E Y
41 M L D L Q P E T T G L Y G Y G Q L N D S
61 S E E E D E I D G P A G Q A E P D R A H
81 Y N I V T F C C K C D S T L R L C V Q S
101 T H V D I R T L E D L L M G T L G I V C
121 P I C S Q K P *
**SEQ ID NO: 36 - ssL2(1-200)**

1 M T Q F N I P V T M S S S L S I I L V I
21 L V S L R T A L S R H K R S A K R T K R
41 A S A T Q L Y K T C K Q A G T C P P D I
61 I P K V E G K T I A E Q I L Q Y G S M G
81 V F F G G L G I G T G S G T G G R T G Y
101 I P L G T R P P T A T D T L A P V R P P
121 L T V D P V G P S D P S I V S L V E E T
141 S F I D A G A P T S V P S I P P D V S G
161 F S I T T S T D T T P A I L D I N N T V
181 T T V T T H N N P T F T D P S V L Q P P
201 T P A E T G G H F T L S S S T I S T H N
221 Y E E I P M D T F *

The attenuated E7GGG gene (E7*)⁷ (which encodes SEQ ID NO:27) was obtained from the E7 gene of HPV16 (HPV16 genome NCBI Reference Sequence: K02718) (which encodes SEQ ID NO:26). The E7*-CP (which encodes SEQ ID NO:33) and the E7*-L-CP (which encodes SEQ ID NO:34) previously constructed by fusing the E7* sequence to the 3' end of the gene of the PVX CP (CP, Nucleotide accession number D00344) (which encodes SEQ ID NO:29) without or with a linker interposition, respectively, were used as templates to obtain the ss-E7* (SEQ ID NO:35), ss-E7*-CP (SEQ ID NO:30)and the ss-E7*-L-CP (SEQ ID NO:31). In these constructs, the E7* gene was N-terminally fused with the PGIP ss from bean (corresponding to the aa sequence MTQFNIPVTMSSSLSIILVILVSLRTALS)¹⁵ (SEQ ID NO:1) by SOE-PCR performed using the Pfu polymerase (Stratagene, La Jolla, CA, USA). The template for PGIP ss was a plant expression vector containing the ss sequence fused to the 3' end of HPV16 E7.⁹

The synthetic sequence L2₁₋₂₀₀-E7* cloned into the pUC vector was already available in the laboratory as a codon-optimized gene for expression in *Chlamydomonas reinhardtii* (purchased by GenScript, Piscataway, NJ, USA). This construct was used as a template to obtain the L2₁₋₂₀₀(which encodes SEQ ID NO:28), the ss-L2₁₋₂₀₀ (which encodes SEQ ID NO:36) and the ss-L2₁₋₂₀₀ -E7* (which encodes SEQ ID NO:32) constructs. L2₁₋₂₀₀ was N-terminally fused with the PGIP ss.

After digestion with either the restriction enzymes KpnI / Notl (for ss-E7*, ss-E7*-CP and ss-E7*-L-CP) or BamHl / Pstl (for L2₁₋₂₀₀, ss-L2₁₋₂₀₀ and ss-L2₁₋₂₀₀-E7*), all fusion fragments were cloned into the expression vector (Fig. 1).

Plasmids integrity was verified by DNA sequencing. In each experiment, all plasmid preparations were purified by CsCI gradient to obtain endotoxin-free products.

### Cell lines

The E7-expressing TC-1 tumor cells were kindly provided by Prof. T.C. Wu (Johns Hopkins Medical Institutions, Baltimore, MD)¹⁶ (Lin KY, Guarnieri FG, Staveley-O'Carroll KF, Levitsky HI, August JT, PardollDM, Wu TC. Treatment of established tumors with a novel vaccine that enhances major histocompatibility class II presentation of tumor antigen. Cancer Res. 1996 Jan 1 ;56(1):21-6.) and were cultured in RPMI with 10% fetal bovine serum (Gibco) supplemented with G418 400 µg/ml. Before inoculation with 5 × 10⁴ cells, TC-1 cells were harvested by trypsinization, washed twice, and resuspended in saline solution.

Human embryonic kidney cells 293 (HEK-293, ATCC^{®} CRL-1573^{™}) were grown in D-MEM (INVITROGEN, Paisley, UK) supplemented with 10% fetal calf serum, 2 mM L-glutamine, 100 IU/ml penicillin and 100 µg/ml streptomycin.

### Mammalian cell transfection, immunofluorescence staining and immunoblot analysis

The expression of the different genes upon transfection was analyzed both by immunoblotting and immunofluorescence.

Transfection of HEK-293 mammalian cells with all the constructs was accomplished with a chemical reagent (Effectene transfection kit; Qiagen) in the presence of 25 µg of MG132 proteasome inhibitor (carbobenzoxy-L-leucyl-L-leucyl-L-leucinal in dimethyl sulfoxide [DMSO]; Calbiochem, Darmstadt, Germany) added to culture medium 24 h post-transfection. Forty-eight hours after transfection, cells were washed twice with ice-cold PBS, scraped, resuspended in gel loading buffer, and boiled for 10 min. Proteins from about 1× 10⁶ cells were analyzed by immunoblotting according to standard procedures. Briefly, lysates were separated by SDS-PAGE and then transferred onto polyvinylidene difluoride (PVDF) membrane.

The immuno-reactive E7* protein in the lysates was analyzed by immunoblotting assay with a mouse polyclonal antibody specific for HPV16 E7 protein (pAb α-E7, gift of P. Di Bonito, National Institute of Health, Rome, Italy) diluted 1:1,000 and, subsequently, with a goat anti-mouse peroxidase-labeled secondary antibody (diluted 1:10,000) purchased by GE Healthcare Life Sciences (Uppsala, Sweden). Bound antibody was detected with the ECL Plus system (GE Healthcare Life Sciences).

To detect the possible secretion of the antigens encoded in the different plasmids provided with the ss, cell culture media were centrifuged at 800 x g to eliminate residual detached cells, ultra-centrifuged at 136.000 x g and concentrated (Millipore centrifugal filter units, with appropriate molecular weight cut off).

For immunofluorescence staining, HEK-293 cells were grown on multi-chamber glass slides and, 48 h after transfection, they were washed twice with ice-cold PBS, fixed with 4% paraformaldehyde for 10 min, and permeabilized with 0.1% Triton X-100 in PBS. Samples were blocked with 5% non-fat dry milk in PBS. E7* and L2₁₋₂₀₀ antigens were stained with either a 1:1,000 diluted mouse pAb α-E7 or a 1:250 diluted mAb RG-1 directed to HPV16 L2 aa 17 to 36 (kind gift of Prof. R. Kirnbauer, Medical University of Wien, Austria)^{5, 17} followed by a 1:300 dilution of an anti-mouse biotinylated secondary antibody (RPN 1021, GE Healthcare Life Sciences, Uppsala, Sweden) and a 1:50 dilution of fluorescein isothiocyanate (FITC)-conjugated streptavidin (Sigma-Aldrich, Steinheim, Germany). Nuclei were counterstained with 4',6-diamidino-2-phenylindole (DAPI) (Sigma-Aldrich, Steinheim, Germany). DAPI emits blue fluorescence upon binding to AT regions of DNA. Slides were examined with a fluorescence Axiolab microscope (Carl Zeiss, Oberkochen, Germany) interfaced with a CoolSNAP CCD camera (Photometrics/Roper Scientific, Tucson, AZ).

### Bacterial expression of the L2₁₋₂₀₀ peptide

The L2₁₋₂₀₀ was expressed in *E*. *coli,* XL-1 blue (Stratagene, San Diego, California), after cloning into the pQE30 vector, and purified both as a soluble His₆-L2₁₋₂₀₀ peptide under native conditions and also under denaturing conditions, by means of Ni-NTA resin. After dialysis against phosphate-buffered saline (PBS), Coomassie blue-stained 12% sodium dodecyl sulfate (SDS)-polyacrylamide gels were used to determine the purity of samples. The amount of immunoreactive His₆-L2₁₋₂₀₀ in the purification fractions was analyzed by immunoblotting using 1:250 mAb RG-1 and, subsequently, with a goat anti-mouse peroxidase-labeled secondary antibody (GE Healthcare Life Sciences, Uppsala, Sweden) diluted 1:10,000).

### Animals and vaccination schedules

Supplied 6-8 week-old female C57BL/6 mice were utilised (Charles River Laboratories srl, Italy). All procedures involving handling and sacrifice of animals were performed under specific pathogen-free conditions at the Animal House of the Regina Elena National Cancer Institute. The study was approved by the Government Committee of National Minister of Health (85/2016-PR) and was conducted according to EU Directive 2010/63/EU for animal experiments.

Two immunization protocols were followed (Fig. 5): the immune response protocol, in which the mice received vaccinations prior immune response analysis, and the therapeutic immunization protocol, in which the mice were challenged with TC-1 tumor cells before administration of the vaccines. In the immunization protocol, animals were primed with the recombinant constructs (100 µg/mouse, IM into the quadriceps), and boosted 2 times at 1 week intervals with the same preparations. In the therapeutic protocol, mice received an injection of 200 µl of saline solution containing 5 × 10⁴ TC-1 tumor cells and, three days after tumor challenge, mice were primed with the same preparations by the same time schedule. Tumor growth was monitored by visual inspection and palpation two times a week. Animals were scored as tumor bearing when tumors reached a size of approximately 1 to 2 mm in diameter.

In a first immunization protocol for the L2-based plasmids, groups of 5 mice were primed with the constructs (100 µg/mouse, IM into the quadriceps) and boosted three times once a week using the same plasmid preparations. In a second immunization protocol performed to improve response,¹⁰ the L2-based plasmids were administered ID in back leg (10 µg/mouse) and IM injection in the other leg (50 µg/mouse into the quadriceps) followed by square wave electroporation at the injection site (100 V of positive and negative polarity at a rate of 1 pulse lasting 50 ms delivered by ECM830 electroporation system BTX Harvard Apparatus tweezer electrodes) using the same plasmid preparations.

### Enzyme-linked immune-sorbent assay (ELISA)

The anti-E7 and the anti-L2₁₋₂₀₀ serum antibodies were determined by a direct ELISA assay. A 96-well plate was coated at 4 °C overnight with 200 ng of either *E. coli*-derived HPV16 His₆-E7 or His₆-L2₁₋₂₀₀ and then blocked with 150 µl of 10% (w/v) no-fat dry milk in PBS. Sera were collected on day 7 after the last boost, diluted in 2% blocking buffer (data are shown for 1:100 dilution), and incubated for 2 h at 37 °C. Total IgGs were detected with horseradish peroxidase (HRP)-conjugated goat anti-mouse IgG(H-L) (GE Healthcare Life Sciences, Uppsala, Sweden).

### IFN-gamma enzyme-linked immuno-spot assay (ELISPOT)

HPV16 E7-specific T cell precursors were detected by enzyme-linked immunospot assay (ELISPOT)¹⁸ one week after the last boost, according to previous reported protocols. A single-cell suspension of splenocytes (1 × 10⁶ cells per well) from each group of vaccinated mice, was added to microtiter wells coated overnight at 4°C with anti-mouse IFN-γ antibody (5µg/ml; BD Biosciences Pharmingen, San Diego, CA), along with interleukin-2 (50 units/ml; Sigma-Aldrich Italia, Milan, Italy). Sample in triplicates were incubated at 37°C for 24-48 hr with the E7-specific H-2Db (10µg/ml) cytotoxic T lymphocyte (CTL) MHC class I epitope (amino acids 49-57, RAHYNIVTF (SEQ ID NO:24) for detecting E7-specific CD8+ T cell precursors. ¹⁹ Plates were incubated with a biotinylated anti-mouse IFN-γ antibody (2 µg/ml) for 4 h at room temperature. Streptavidin-HRP was then used for 1 h at room temperature, and the cell spots stained by addition of the filtered 3-amino-9-ethylcarbazole substrate (BD Biosciences Pharmingen, San Diego, CA), for 1-5 min. The reaction was terminated once the formation of discrete purple-colored spots was detected. Spots were counted using a dissecting microscope.

### Statistical analysis

Statistical analyses of ELISA data were performed using the one-way ANOVA parametric test and Bonferroni's analysis of variance, using Graph Pad Prism 5 software (www.graphpad.com). The statistical significance was set as p< 0,05.

### Results

### Expression of the ss-provided HPV genes in transfected HEK-293 cells.

HEK-293 cells were transiently transfected to study the expression and the intracellular fate of the proteins encoded by the DNA constructs ss-E7GGG, ss-E7GGG -CP and ss-E7GGG -L-CP (Fig.1) in a mammalian cell model. The ss-E7GGG construct was expressed in HEK-293 and found in the culture medium of transfected cells, whereas E7GGG without ss was only present in the cell lysates, as shown by immunoblotting analysis (Fig. 2A). The immunofluorescence analysis revealed a specific FITC fluorescence (green) exclusively associated to the cytosol and probably to intra-cellular membranes for ss-E7GGG (Fig. 2B), while E7GGG without ss was detected mainly in the nucleus (Fig. 2C).

For the ss-E7GGG-CP and E7GGG-CP constructs, expression but no secretion was observed in the culture medium. The lysates of the cells transfected with ss-provided constructs, showed presence of a double band (Fig. 3A). In the presence of ss, a specific green fluorescence that seemed to be associated to intra-cellular membranes, was observed especially for ss-E7GGG-CP (Fig.3C). As already reported,⁷ the fusion of E7 GGG with the CP sequence determines the re-distribution and aggregation of the E7GGG protein into the cytoplasm (Fig. 3B, 3D).

The L2 constructs were also expressed and proteins detected in the transfected cells mostly into the cytoplasm (Fig 4A, B, C).

*Mouse immunological responses to vaccination with ss-E7GGG, ss-E7* GGG-*CP and ss-E7GGG-L-CP plasmids.*

The immunological effects of the ss-provided DNA vaccines were studied in C57BL/6 mice with the prime/boost schedule described in figure 5A.

The effectiveness of the ss-E7GGG-based DNA vaccines in inducing cell-mediated responses was studied in ELISPOT assay for INF-γ secreting cells. One week after TC-1 challenge, spleens were collected from immunized mice. By the addition of ss, no enhancement of the cell-mediated immune response specifically directed against E7 was observed with respect to the non-secretory constructs. Actually, the scores obtained with the ss-series of plasmids were inferior to those recorded in mice vaccinated with the plasmids without ss. In brief, both ss-E7GGG-CP and ss-E7GGG-L-CP vaccination elicited an E7-specific CTL response approximately a 3-fold lower than E7GGG-CP, used for comparison. The lowest score was obtained in ss- E7GGG vaccinated mice, exhibiting E7-specific T cell response approximately a 7-fold lower than that recorded in mice vaccinated with E7GGG-CP (Fig. 6).

E7 specific serum antibody response was evaluated one week after the boost. Circulating antibodies were detected in ELISA assay with *E*. *coli*-produced E7 protein. Total serum specific IgGs were induced after immunization with ss-E7GGG and ss-E7GGG -CP. This anti-E7 response was significantly higher than that of DNA vaccines without ss (Fig.7) with means and variances significantly different (pmeans< 0,05 and pvariances< 0,05).

### Therapeutic immunization with ss-E7*, ss-E7*-CP and ss-E7*-L-CP plasmids after challenge with TC-1-induced tumors in mice.

The peculiar immunological responses of these new anti-E7 vaccines led us to determine if a therapeutic potential of the DNA vaccines could still be envisaged in the TC-1 pre-clinical model. Indeed, E7 is a target for the development of therapeutic candidate HPV vaccines. The E7*-CP vaccine was utilized for comparison. After injecting TC-1 cells into naïve C57BL/6 mice, prime and boost immunizations were performed on days 3 and 10, respectively (Fig. 5B). As shown in figure 8, despite tumor appearance was delayed to day 30 post challenge in all anti-E7 immunized mice, at day 47, 67% of the E7*-CP treated animals was still tumor-free, while the ss-constructs induced a weaker effect: 33% of ss-E7*-L-CP vaccinated mice and 17% of ss-E7* or ss-E7*-CP vaccinated mice were tumor free, respectively. By the same day, 100% of the mice immunized with the empty vector developed tumors.

### Mouse immunological responses to vaccination with L2-based DNA constructs

The structural conservation of L2 makes this capsid protein a target for the development of 'pan-HPV' vaccines. Based on this consideration, the plant ss was genetically fused to a cross-reactive long peptide of the L2 protein (L2₁₋₂₀₀, about 24 kDa) to produce a broad range preventive HPV vaccine. In addition, to investigate for a therapeutic/preventive HPV vaccine, fusions of L2₁₋₂₀₀ were made with the E7* sequence. C57BU6 mice were immunized according the two different protocols described in Material and Methods (Fig 5C, 5D).

The L2₁₋₂₀₀ expressed and purified from *E*. *coli* (Fig. 9) was used to set-up ELISA test for antigen-specific IgG responses. Data for ELISA test were generated from sera collected from each mouse of every group and vaccination experiments were performed three times with similar results. One representative experiment is shown in figure 10. The 'long-term' immunization protocol (Fig. 5C) was not able to induce a significant improvement of anti-L2₁₋₂₀₀ antibody titers compared to pre-immune sera (data not shown). When the vaccination schedule implying electroporation (EP, Fig. 5C) was applied, animals were primed with DNA at time zero and boosted with DNA at 1-week interval. The repeated vaccination with the plasmids delivered by intradermal (ID) and EP immunization within a short interval, resulted in the induction of a strong humoral immune response (Fig. 10). The ss-provided constructs induced a statistically significant higher response against both L2₁₋₂₀₀ and E7 compared to DNA vaccines without ss (pₘₑₐₙₛ< 0,05 and p_{variances}< 0,05). The ss-L2₁₋₂₀₀-E7* plasmid was able to produce the highest titers of both anti-L2₁₋₂₀₀ (Fig. 10A) and anti-E7 IgG (Fig. 10B). The EP immunization protocol determined also a long-lasting maintenance of the acquired immunity as shown by the anti-L2 reactivity of the pooled sera collected from mice 6 months after immunization (Fig. 11).

### References

1. Vici P, Mariani L, Pizzuti L, Sergi D, Di Lauro L, Vizza E, Tomao F, Tomao S, Mancini E, Vincenzoni C, et al. Emerging biological treatments for uterine cervical carcinoma. J Cancer 2014; 5:86-97.
2. Mariani L, Venuti A. HPV vaccine: an overview of immune response, clinical protection, and new approaches for the future. J Transl Med 2010; 8:105.
3. Tyler M, Tunban E, Chackerian B. Second-generation prophylactic HPV vaccines: successes and challenges. Expert Rev Vaccines 2014;13:247-55.
4. Whang DW, Zhihai L, Jieqiong X, Junqi W, Li Z, Yajing L, Fei F, Lu X, Minxi W, Zhibo K, et al. Identification of broad-genotype HPV L2 neutralization site for pan-HPV vaccine development by a cross-neutralizing antibody. PLoS One 2015;10:1-18.
5. Ghambira R, Karanam B, Jagu S, Roberts JN, Buck CB, Bossis I, Alphs H, Culp T, Christensen ND, Roden RB. A protective and broadly cross-neutralizing epitope of human papillomavirus L2. J Virol 2007;81:13927-31.
6. Trimble CL, Morrow MP, Kraynyak KA, Shen X, Dallas M, Yan J, Edwards L, Parker RL, Denny L, Giffear M, Brown AS, Marcozzi-Pierce K, Shah D, Slager AM, Sylvester AJ, Khan A, Broderick KE, Juba RJ, Herring TA, Boyer J, Lee J, Sardesai NY, Weiner DB, Bagarazzi ML. Safety, efficacy, and immunogenicity of VGX-3100, a therapeutic synthetic DNA vaccine targeting human papillomavirus 16 and 18 E6 and E7 proteins for cervical intraepithelial neoplasia 2/3: a randomised, double-blind, placebo-controlled phase 2b trial. Lancet. 2015 Nov 21;386(10008):2078-88.
7. Massa S, Simeone P, Muller A, Benvenuto E, Venuti A, Franconi R. Antitumor activity of DNA vaccines based on the human papillomavirus-16 E7 protein genetically fused to a plant virus coat protein. Hum Gene Ther 2008;19:354-64.
8. Noemi 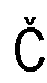 eřovská, Hana Hoffmeisterová, Tamara Pe 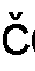 enková, Tomá 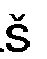 Moravec, Helena Synková, Helena Plchová, Jiří Velemínský. Transient expression of HPV16 E7 peptide (aa 44-60) and HPV16 L2 peptide (aa 108-120) on chimeric potyvirus-like particles using Potato virus X-based vector. Protein Expression and Purification Academic Press, San Diego. CA, Volume 58, no. 1, January 2008, Pages 154-161.
9. Franconi R, Massa S, Illiano E, Muller A, Cirilli A, Accardi L, Di Bonito P, Giorgi C, Venuti A. Exploiting the plant secretory pathway to improve the anti-cancer activity of a plant-based HPV16 E7 vaccine. International J Immunopathol Pharmacol 2006;19:785-95.
10. Hallengärd D, Haller BK, Maltais A-K, Gelius E, Nihlmark, Wahren B, Bråve1 A. Comparison of plasmid vaccine immunization schedules using intradermal in vivo electroporation. Clin Vaccine Immunol 2011;18:1577-81.
11. Low L, Mander A, McCann K, Dearnaley D, Tjelle T, Mathiesen I, Stevenson F, Ottensmeier CH. DNA vaccination with electroporation induces increased antibody responses in patients with prostate cancer. Hum Gene Ther 2009;20:1269-78.
12. Vasan S, Hurley A, Schlesinger SJ, Hannaman D, Gardiner DF, Dugin DP, Boente-Carrera M, Vittorino R, Caskey M, Andersen J. et al. In vivo electroporation enhances the immunogenicity of a HIV-1 DNA vaccine candidate in healthy volunteers. PLoS One 2011 ;6:e19252.
13. Von Heijne G. The signal peptide. J Membrane Biol. 1990;115:195-201.
14. Güler-Gane G, Kidd S, Sridharan S, Vaughan TJ, Wilkinson TCI, Tigue NJ. Overcoming the refractory expression of secreted recombinant proteins in mammalian cells through modification of the signal peptide and adjacent amino acids. PLoS One 2016;11 (5):e0155340.
15. Toubart P, Desiderio A, Salvi G, Cervone F, Daroda L, De Lorenzo G, Bergmann C, Darvill AG, Albersheim P. Cloning and characterization of the gene encoding the endopolygalacturonase-inhibiting protein (PGIP) of Phaseolus vulgaris L. Plant J 1992;2: 367-73.
16. Lin KY, Guarnieri FG, Staveley-O'Carroll KF, Levitsky HI, August JT, PardollDM, Wu TC. Treatment of established tumors with a novel vaccine that enhances major histocompatibility class II presentation of tumor antigen. Cancer Res. 1996 Jan 1;56(1):21-6.
17. Kirnbauer Schellenbacher C, Roden R, Kirnbauer R. Chimeric L1-L2 virus-like particles as potential broad-spectrum human papillomavirus vaccines. J Virol 2009;83:10085-95.
18. Miyahira Y, Murata K, Rodriguez D, Rodriguez JR, Esteban M, Rodrigues MM, Zavala F. Quantification of antigen specific CD8+ T cells using an ELISPOT assay. J Immunol Methods 1995;181 :45-54.
19. Hsu KF, Hung CF, Cheng WF, He L, Slater LA, Ling M, Wu TC. Enhancement of suicidal DNA vaccine potency by linking Mycobacterium tuberculosis heat shock protein 70 to an antigen. Gene Ther 2001 ;8:376-83.

## Claims

1. DNA vaccine comprising a polynucleotide sequence which encodes a fusion protein or an expression vector or plasmid comprising said polynucleotide sequence, said fusion protein being SEQ ID NO:32.

2. DNA vaccine according to claim 1 that is a pharmaceutical composition which further comprises one or more excipients and/or adjuvants.

3. DNA vaccine according to anyone of the previous claims, wherein the DNA vaccine is administered by electroporation.

4. DNA vaccine as defined in anyone of claims 1-3, for use in the prevention and treatment of HPV etiology cancer.

5. DNA vaccine as defined in anyone of claims 1-3, for use according to claim 4, wherein the DNA vaccine is administered, in a preferred way, by electroporation.

6. Process for the preparation of the DNA vaccine as defined in anyone of claims 1-3, said process comprising a) preparing a fusion gene encoding the fusion protein of sequence SEQ ID NO:32 by fusing a nucleotide sequence encoding the plant protein signal sequence of polygalacturonase-inhibiting protein, said plant protein signal sequence having sequence MTQFNIPVTMSSSLSIILVILVSLRTALS (SEQ ID NO:1), to the N-terminal portion of a nucleotide sequence encoding an antigenic sequence consisting of the immunogenic fragment consisting of aminoacids 1-200 of L2 of HPV16 and E7GGG of HPV16; b) cloning the fusion gene in a plasmid vector for expression in mammalian cells, such as pcDNA3.1, pVAX; and c) amplification of the plasmid vector in a suitable bacterial microorganism, such as *E*. *coli,* and isolation thereof from the microorganism.

## Patentansprüche

1. DNA-Impfstoff, eine Polynukleotidsequenz umfassend, die ein Fusionsprotein oder einen Expressionsvektor oder ein Plasmid enthält, welches die besagte Polynukleotidsequenz umfasst, wobei das besagte Fusionsprotein SEQ ID NO:32 ist.

2. DNA-Impfstoff nach Anspruch 1, der eine pharmazeutische Zusammensetzung ist, welche ferner einen oder mehrere Begleit- und/oder Hilfsstoffe umfasst.

3. DNA-Impfstoff nach einem der vorhergehenden Ansprüche, wobei der DNA-Impfstoff durch Elektroporation verabreicht wird.

4. DNA-Impfstoff wie in einem der Ansprüche 1-3 definiert, zur Verwendung in der Vorbeugung und Behandlung von HPV-Ätiologie-Krebs.

5. DNA-Impfstoff wie in einem der Ansprüche 1-3 definiert, zur Verwendung gemäß Anspruch 4, wobei der DNA-Impfstoff vorzugsweise durch Elektroporation verabreicht wird.

6. Verfahren zur Herstellung des DNA-Impfstoffs wie in einem der Ansprüche 1-3 definiert, wobei das Verfahren umfasst: a) Herstellen eines Fusionsgens, welches das Fusionsprotein der Sequenz SEQ ID NO:32 kodiert, durch Fusionieren einer Nukleotid-Sequenz, welche die Pflanzenprotein-Signalsequenz des Polygalacturonaseinhibierenden Proteins kodiert, die besagte Pflanzenprotein-Signalsequenz hat die Sequenz MTQFNIPVTMSSSLSIILVILVSLRTALS (SEQ ID NO:1), zum N-terminalen Teil einer Nukleotidsequenz, die eine Antigen-Sequenz kodiert, bestehend aus dem immunogenen Fragment, das aus Aminosäuren 1-200 von L2 von HPV16 und E7GGG von HPV16 besteht; b) Klonen des Fusionsgens in einem Plasmidvektor zur Expression in Zellen von Säugetieren, wie z.B. pcDNA3.1, pVAX; und c) Amplifikation des Plasmidvektors in einem geeigneten bakterieller Mikroorganismus, wie *E. coli,* und Isolierung davon aus dem Mikroorganismus.

## Revendications

1. Vaccin à ADN comprenant une séquence polynucléotidique qui code une protéine de fusion ou un vecteur d'expression ou un plasmide comprenant ladite séquence polynucléotidique, ladite protéine de fusion étant SEQ ID NO:32.

2. Vaccin à ADN selon la revendication 1 qui est une composition pharmaceutique
qui comprend en outre un ou plusieurs excipients et/ou substances auxiliaires.

3. Vaccin à ADN selon l'une quelconque des revendications précédentes, dans lequel le vaccin à ADN est administré par électroporation.

4. Vaccin à ADN tel que défini dans l'une quelconque des revendications 1 à 3, destiné à être utilisé dans la prévention et le traitement du cancer de l'étiologie du VPH.

5. Vaccin à ADN tel que défini dans l'une quelconque des revendications 1 à 3, destiné à être utilisé suivant la revendication 4, dans laquelle le vaccin à ADN est administré, préférablement, par électroporation.

6. Procédé de préparation du vaccin à ADN tel que défini dans l'une quelconque des revendications 1 à 3, ledit procédé comprenant a) la préparation d'un gène de fusion codant la protéine de fusion de séquence SEQ ID NO:32 en fusionnant une séquence nucléotide codant la séquence signal de la protéine végétale de protéine inhibant la polygalacturonase, ladite séquence signal de protéine végétale ayant la séquence MTQFNIPVTMSSSLSIILVILVSLRTALS (SEQ ID NO:1), à la partie N-terminale d'une séquence nucléotidique codant une séquence antigénique constituée du fragment immunogène qui consiste des acides aminés 1-200 de L2 de HPV16 et E7GGG de HPV16 ; b) cloner le gène de fusion dans un vecteur plasmidique pour l'expression dans des cellules de mammifères, tel que pcDNA3.1, pVAX; et c) amplification du vecteur plasmidique dans un micro-organisme bactérien approprié, tel que *E. coli,* et isolement de celui-ci du micro-organisme.
